# EUROPEAN PATENT APPLICATION

(11) **EP 1 867 641 A1**
(43) Date of publication of application: **19.12.2007**
(21) Application number: 06730995.5
(22) Date of filing: 03.04.2006
(51) Int. Cl.: C07D 307/77, A61K 31/343, A61K 45/00, A61P 25/20, A61P 25/22, A61P 25/24, A61P 25/28, A61P 43/00

(54) **PREVENTIVE OR REMEDY FOR DEPRESSION OR ANXIETY NEUROSIS**

(30) Priority: 04.04.2005 JP 2005107674
(71) Applicant: Takeda Pharmaceutical Company Limited, Osaka-shi, Osaka 541-0045 (JP)
(72) Inventor: HIRAI, Keisuke, TAKEDA PHARMACEUTICAL COMPANY LTD., Osaka-shi, Osaka 5328686 (JP); MIYAMOTO, Masaomi, TAKEDA PHARMACEUTICAL CO. LTD., Osaka-shi, Osaka 5328686 (JP)
(74) Representative: Rickard, Timothy Mark Adrian
(86) International application number: PCT/JP2006/307047
(87) International publication number: WO 2006/107019

(57) **Abstract**

The present invention provides a prophylactic or therapeutic agent for depression or anxiety disorders, comprising (S)-N-[2-(1,6,7,8-tetrahydro-2H-indeno[5,4-b]furan-8-yl)ethyl]propionamide.

## Description

### Technical Field

The present invention relates to a prophylactic or therapeutic agent for depression or anxiety disorders.

### Background Art

(S)-N-[2-(1,6,7,8-Tetrahydro-2H-indeno[5,4-b]furan-8-yl)ethyl]propionamide (generic name: ramelteon, hereinafter, sometimes referred to as compound A) has a melatonin agonistic action, and is a known therapeutic agent for sleep disorders, which is disclosed in patent document 1.
[patent document 1] US patent No. 6,034,239

### Disclosure of Invention

### Problems to be Solved by the Invention

The object of the present invention is to provide a prophylactic or therapeutic agent for depression or anxiety disorders which is low toxic.

### Means of Solving the Problems

As a result of intensive studies, the present inventors have found out that compound A, that is, (S)-N-[2-(1,6,7,8-tetrahydro-2H-indeno[5,4-b]furan-8-yl)ethyl]propionamide is effective for prevention or treatment of depression or anxiety disorders, and completed the present invention.

That is, the present invention provides:
[1] A pharmaceutical composition for prevention or treatment of depression or anxiety disorders, which comprises (S)-N-[2-(1,6,7,8-tetrahydro-2H-indeno[5,4-b]furan-8-yl)ethyl]propionamide;
[2] A pharmaceutical composition for prevention or treatment of depression or anxiety disorders, which comprises combining (S)-N-[2-(1,6,7,8-tetrahydro-2H-indeno[5,4-b]furan-8-yl)ethyl]propionamide and one or more drugs selected from other antidepressants and antianxiety drugs;
[3] A pharmaceutical composition for prevention or treatment of depression or anxiety disorders, which comprises (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam;
[4] The pharmaceutical composition for prevention or treatment of depression or anxiety disorders according to any one of the above-mentioned [1] to [3], which is a prophylactic or therapeutic agent for depression or anxiety disorders of patients having a background of diabetes, hyperlipidemia, hypertension or metabolic syndrome;
[5] A method for preventing or treating depression or anxiety disorders, which comprises administering (S)-N-[2-(1,6,7,8-tetrahydro-2H-indeno[5,4-b]furan-8-yl)ethyl]propionamide;
[6] A method for preventing or treating depression or anxiety disorders of patients having a background of diabetes, hyperlipidemia, hypertension or metabolic syndrome, which comprises administering (S)-N-[2-(1,6,7,8-tetrahydro-2H-indeno[5,4-b]furan-8-yl)ethyl]propionamide;
[7] A method for preventing or treating depression or anxiety disorders, which comprises administering (S)-N-[2-(1,6,7,8-tetrahydro-2H-indeno[5,4-b]furan-8-yl)ethyl]propionamide and one or more drugs selected from other antidepressants and antianxiety drugs;
[8] A method for preventing or treating depression or anxiety disorders of patients having a background of diabetes, hyperlipidemia, hypertension or metabolic syndrome, which comprises administering (S)-N-[2-(1,6,7,8-tetrahydro-2H-indeno[5,4-b]furan-8-yl)ethyl]propionamide in combination with one or more drugs selected from other antidepressants and antianxiety drugs;
[9] A method for preventing or treating depression or anxiety disorders, which comprises administering (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam;
[10] A method for preventing or treating depression or anxiety disorders of patients having a background of diabetes, hyperlipidemia, hypertension or metabolic syndrome, which comprises administering (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam;
[11] Use of (S)-N-[2-(1,6,7,8-tetrahydro-2H-indeno[5,4-b]furan-8-yl)ethyl]propionamide for manufacturing a pharmaceutical composition for prevention or treatment of depression or anxiety disorders;
[12] Use of (S)-N-[2-(1,6,7,8-tetrahydro-2H-indeno[5,4-b]furan-8-yl)ethyl]propionamide for manufacturing a pharmaceutical composition for prevention or treatment of depression or anxiety disorders of patients having a background of diabetes, hyperlipidemia, hypertension or metabolic syndrome;
[13] Use of (S)-N-[2-(1,6,7,8-tetrahydro-2H-indeno[5,4-b]furan-8-yl)ethyl]propionamide in combination with one or more drugs selected from other antidepressants and antianxiety drugs for manufacturing a pharmaceutical composition for prevention or treatment of depression or anxiety disorders;
[14] Use of (S)-N-[2-(1,6,7,8-tetrahydro-2H-indeno[5,4-b]furan-8-yl)ethyl]propionamide in combination with one or more drugs selected from other antidepressants and antianxiety drugs for manufacturing a pharmaceutical composition for prevention or treatment of depression or anxiety disorders of patients having a background of diabetes, hyperlipidemia, hypertension or metabolic syndrome;
[15] Use of (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam for manufacturing a pharmaceutical composition for prevention or treatment of depression or anxiety disorders;
[16] Use of (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam for manufacturing a pharmaceutical composition for prevention or treatment of depression or anxiety disorders of patients having a background of diabetes, hyperlipidemia, hypertension or metabolic syndrome; and the like.

According to the present invention, a prophylactic or therapeutic agent for depression or anxiety disorders which is low toxic is provided.

### Brief Description of Drawings

Fig. 1 is a graph of lick frequency in water-drinking conflicting test.
Fig. 2 is a graph of shock frequency in water-drinking conflicting test.
Fig. 3 is a graph of lick frequency in water-drinking conflicting test.
Fig. 4 is a graph of shock frequency in water-drinking conflicting test.
Fig. 5 is a graph of time spent in open arms of elevated plus-maze.
Fig. 6 is a graph of number of entries into open arms of elevated plus-maze.
Fig. 7 is a graph of the effect of each of Paroxetine and Compound A on time-course of immobility time.
Fig. 8 is a graph of the effect of each of Paroxetine and Compound A on immobility time.
Fig. 9 is a graph of the effect of each of Paroxetine and Compound A on moving power.
Fig. 10 is a graph of the effect of each of Paroxetine and Compound A on moving power.
Fig. 11 is a graph of the effect of combination of Paroxetine and Compound A on immobility time.
Fig. 12 is a graph of the effect of combination of Paroxetine and Compound A on immobility time.
Fig. 13 is a graph of the effect of combination of Paroxetine and Compound A on moving power.
Fig. 14 is a graph of the effect of combination of Paroxetine and Compound A on moving power.

### Best Mode for Carrying Out the Invention

(S)-N-[2-(1,6,7,8-Tetrahydro-2H-indeno[5,4-b]furan-8-yl)ethyl]propionamide, that is, compound A to be used in the present invention is a known therapeutic agent for sleep disorders disclosed in US 6,034,239 etc., and can be produced by a known method such as that disclosed in said reference.

Compound A has an antidepressant and antianxiety action. Therefore, compound A can be used for preventing or treating depression or anxiety disorders.

In addition, since along with the antidepressant and antianxiety action, compound A inhibits diabetes and hyperlipidemia, and, or improves metabolic syndromes such as hypertension, it is particularly effective for the prevention or treatment of depression or anxiety disorders of patients having a background of diabetes, hyperlipidemia, hypertension or metabolic syndrome.

Further, since compound A is extremely low toxic, it can be used for a prevention or treatment of depression or anxiety disorders in combination with other antidepressants and antianxiety drugs, and then the side effects caused by the other antidepressants and antianxiety drugs can be reduced by lowering the dose of such drugs. Besides, compound A has an advantage of hardly aggravating side effects of the other antidepressants and antianxiety drugs used in combination with compound A.

Examples of such antidepressants include tricyclic antidepressants [e.g., Doxepin, Imipramine hydrochloride, Amitriptyline, Clomipramine], tetracyclic antidepressants [e.g., Mianserine, Setiptiline, Maprotiline], SSRI [e.g., Fluoxetine, Sertraline, Paroxetine, Citalopram, Escitalopram, Fluvoxamine], SNRI [e.g., Milnacipran, Duloxetine, Venlafaxine, Trazodone, Nefazodone, Minaprine, Mirtazapine, Buspirone], NKI antagonist, drugs having both melatonin agonistic action and serotonin II antagonistic action [e.g., Agomelatine], and the like.

Examples of such antianxiety drugs include benzodiazepine antianxiety drugs [e.g., Diazepam, Flutazolam, Lorazepam, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate, Oxazolam], serotonin antianxiety drugs [e.g., Buspirone, Tandospirone], and the like.

These antidepressants and antianxiety drugs may be a free form or a pharmaceutically acceptable salt. In case that the antidepressants and antianxiety drugs have an acidic functional group, examples of the salts include inorganic salts such as alkali metal salts (e.g., sodium salt, potassium salt, etc.), alkaline earth metal salts (e.g., calcium salt, magnesium salt, barium salt, etc.), ammonium salt, and the like. In addition, in case that the antidepressants and antianxiety drugs have a basic functional group, examples of the salts include salts with inorganic acid such as hydrochloric acid, hydrobromic acid, nitric acid, sulfuric acid, phosphoric acid, and the like, and salts with an organic acid such as acetic acid, phthalic acid, fumaric acid, oxalic acid, tartaric acid, maleic acid, citric acid, succinic acid, methanesulfonic acid, p-toluenesulfonic acid, and the like. The known antidepressants and antianxiety drugs exemplified here can be commercially available with ease, or can be produced according to a known method.

When compound A is used in combination with other antidepressants or antianxiety drugs, examples of administration forms include (1) administration of a single preparation obtained by formulating compound A and other antidepressant or antianxiety drug simultaneously, (2) simultaneous administration of two preparations obtained by formulating compound A and other antidepressant or antianxiety drug separately, via an identical route, (3) sequential and intermittent administration of two preparations obtained by formulating compound A and other antidepressant or antianxiety drug separately, via an identical route, (4) simultaneous administration of two preparations obtained by formulating compound A and other antidepressant or antianxiety drug separately, via different routes, (5) sequential and intermittent administration of two preparations obtained by formulating compound A and other antidepressant or antianxiety drug separately, via different routes (e.g. administration in the order of compound A → other antidepressant or antianxiety drug, or in the inverse order) and the like. From a viewpoint of convenience of patients, preferred is an administration of a single preparation obtained by formulating compound A and other antidepressant or antianxiety drug simultaneously.

The dosage of the combined drug can be appropriately selected based on a clinically used dose. In addition, the blending ratio of compound A and other antidepressant or antianxiety drug can be appropriately selected depending on administration subject, administration route, target disease, symptom, other antidepressant or antianxiety drug to be used and the like. Usually, the ratio may be decided based on the general dose of the other antidepressant or antianxiety drug to be used. When the administration subject is human, for example, 0.01-100 parts by weight of the other antidepressant or antianxiety drug is used relative to 1 part by weight of compound A.

Compound A can be safely administered orally or parenterally (e.g. topically, rectally, intravenously etc.) as it is or as a pharmaceutical composition mixed with pharmacologically acceptable carriers according to a conventional method (e.g., method described in Japanese Pharmacopoeia, etc.), such as tablets (including sugar-coated tablets, film-coated tablets), powders, granules, capsules, solutions, emulsions, suspensions, injectables, suppositories, sustained-release agents, adhesive preparations, and the like.

The content of compound A is usually about 0.01 to 100% by weight based on a total weight of the composition.

The dose of compound A differs depending on an administration subject, administration route, and disease. For example, when administered to an adult as an oral agent, the dosage is about 0.0005 to 2 mg/kg body weight, preferably about 0.001 to 1 mg/kg body weight, more preferably about 0.001 to 0.5 mg/kg body weight in terms of compound (I) as an active ingredient. The pharmaceutical composition may be administered once to several times in divided doses per day.

The present invention will be described in detail through the following Examples and Preparation Examples. However, these are just an example, and the present invention is not limited by the examples, and may be changed without departing from the scope of the present invention.

### [Examples]

### Example 1 (water-drinking conflicting test)

Animal: male Wister (Jcl) rats were purchased at the age of 8 weeks old, and used at the age of 9 weeks old.

First, rats were put in a separate cage, and abstained from water for two days under light-dark (12-hour cycle) condition. On the day of experiment, rats were habituated to the experiment room for more than 60 minutes, and administered intraperitoneally with 30 mg/kg of compound A, 30 mg/kg of melatonin or vehicle, and then test was carried out 30 minutes after the administration.

The drug used (compound A, melatonin) was suspended in 0.5% methylcellulose physiological saline to adjust dosage to 2 mL/kg, and administered intraperitoneally. To the vehicle administration group, a 0.5% methylcellulose physiological saline was administered in the same way. All administrations and trials were performed between 9 : 00 and 12 : 00.

In the present experiment, when the animals pre-raised under a water-deprived condition drink water, they are placed in a conflicting state that they get electroshocked under the following condition. In addition, it is confirmed that as a control drug, Diazepam which is a commercial antianxiety drug exhibits an effect in this system.

### [Measurement Condition]

Test Time: 300 seconds, Shock Condition: in case either 20 times of lick frequency or 2 seconds of lick time is satisfied
Shock ON Time: 2 seconds, Time-out: 180 seconds, Stimulus Intensity: 0.6 mA

### [Result]

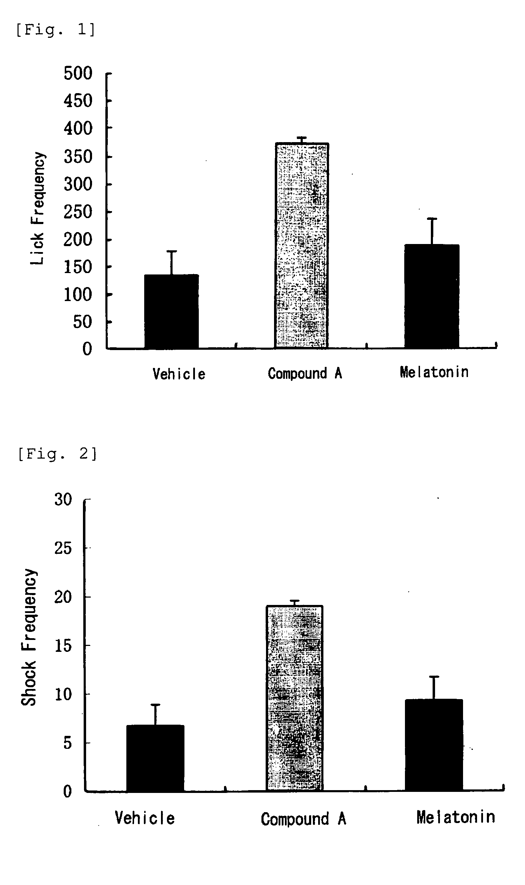

Williams' test was used for determining statistical significance. As shown in Figs. 1 and 2, in the comparison with vehicle administration group, a significant increasing effect of lick frequency and shock frequency was observed in compound A administration group. In addition, in the melatonin administration group, a little anti-conflicting effect was observed, but it was not a significant effect. From the result of this test, it was shown that compound A has an antidepressant effect and antianxiety effect. On the other hand, unexpectedly, melatonin did not show such effect.

### Example 2 (water-drinking conflicting test)

Animal: male Wister (Jcl) rats were purchased at the age of 8 weeks old, and used at the age of 9 weeks old.
Drug Administration Group and Test Method: Test was carried out for the following conditions. (each group n = 10)
1. Vehicle (0.5% MC)
2. Diazepam 0.3 mg/kg
3. Diazepam 0.3 mg/kg + Compound A 0.3 mg/kg
4. Diazepam 0.3 mg/kg + Compound A 10 mg/kg
5. Diazepam 0.3 mg/kg + Compound A 30 mg/kg
6. Compound A 30 mg/kg

Compound A and Diazepam were suspended in 0.5% methylcellulose solution to adjust dosage to 2 mL/kg, and administered intraperitoneally. To control group, a 0.5% methylcellulose physiological saline for injection was administered as vehicle. As a drug solution to be combined, a drug solution having twice concentration of final concentration was prepared, and adjusted to final concentration by mixing equal amounts. A dose of 2 mL/kg was administered intraperitoneally, respectively. All administrations and trials were performed between 9 : 00 and 13 : 00.

First, rats were put in a separate cage, and abstained from water for two days under light-dark (12-hour cycle) condition. On the day of experiment, rats were habituated to the experiment room for more than 60 minutes, and each drug was administered intraperitoneally, and then test was carried out 30 minutes after the administration.

### [Measurement Condition]

Test Time: 300 seconds, Shock Condition: in case either 20 times of lick frequency or 2 seconds of lick time is satisfied
Shock ON Time: 2 seconds, Time-out: 180 seconds, Stimulus Intensity: 0.6 mA

### [Result]

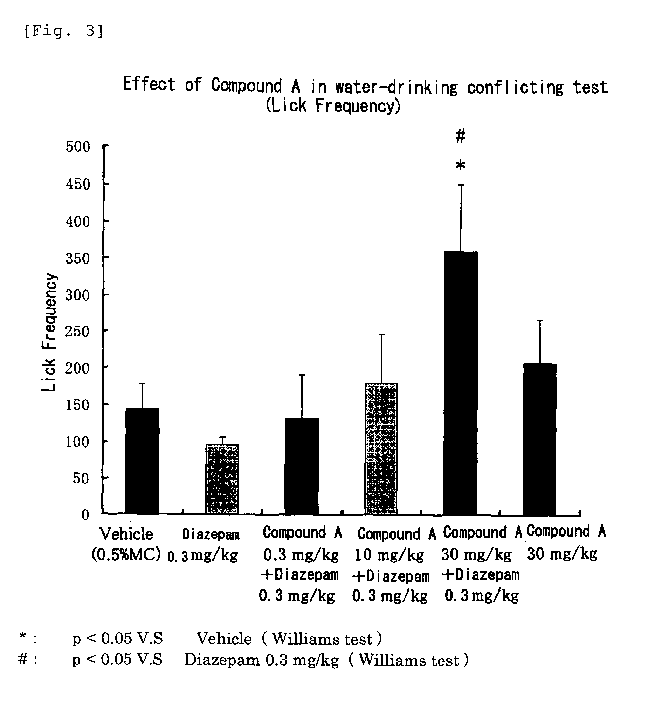

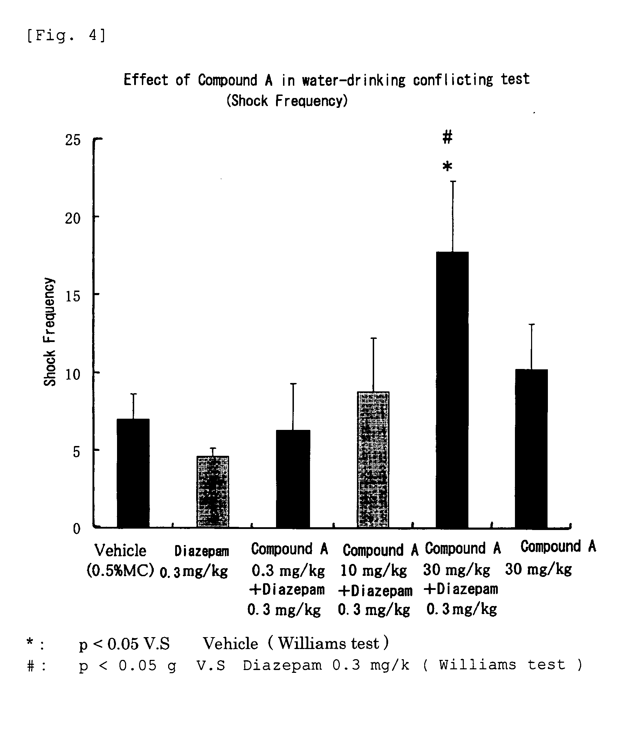

Significant difference test was performed with using Williams' test for vehicle administration group and multigroup drug administration groups. t-Test was used for vehicle administration group and single drug administration groups. A dose of compound A (0.3, 10 and 30 mg/kg) and 0.3 mg/kg of Diazepam were administered in combination. As shown in Figs. 3 and 4, a significant increase in lick frequency and shock frequency was observed with the dose of 30 mg/kg of compound A combined with 0.3 mg/kg of diazepam in the comparison with vehicle and 0.3 mg/kg of diazepam administration groups, respectively.

### Example 3 (elevated plus-maze)

Animal: male Wister (Jcl) rats were purchased at the age of 5 weeks old, and used at the age of 6 weeks old.

An elevated plus-maze test equipment having arm 50 cm long by 10 cm wide was used. Its height was 40 cm, and the height of closed arm was adjusted to the same. Black was used as the color of wall. Lighting in experiment room was set to 5 lux of illuminance on the equipment.

The animals were handled from 2 days before test. Test was carried out between 8 a.m. and 13 p.m., and the animals were naturalized in the conduct test room at a measurement illuminance from 1 hour before the start of test.

The drug used (compound A, melatonin) was suspended in 0.5% methylcellulose physiological saline to adjust dosage to 2 mL/kg, and administered intraperitoneally. To the vehicle administration group, a 0.5% methylcellulose physiological saline was administered in the same way.

In the elevated plus-maze test, the animal was put in the central region of maze with the head to the direction of closed arm, and the conduct of 5 minutes duration was observed. Measurement items were number of entries into open arms and time spent in open arms which were used as an indicator of anxiety conducts, and it was considered that the more the number of entries into open arms, time spent in open arms and dipping frequency are, the less the anxiety conducts are. Further, the number of entries into closed arms was counted, which was used as an indicator of amount of motor-activity. In addition, it is confirmed that as a control drug, Diazepam which is a commercial antianxiety drug exhibits an effect in this system. Test was performed with n = 10 for each group. Trial was carried out over 2 days, and 2 days' data were summed up as one test.

### [Result]

Significant difference test was performed with using Williams' test for vehicle administration group and multigroup drug administration groups.

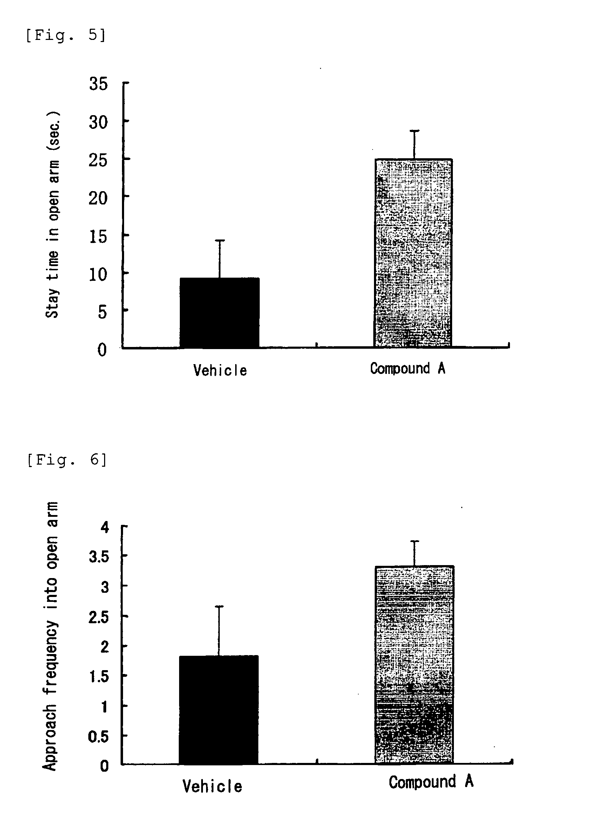

As shown in Figs. 5 and 6, in the comparison with vehicle administration group, an increase in the number of entries into open arms and time spent there were observed in compound A administration group. In addition, the number of entries into closed arms which provides an indicator of amount of motor-activity remained almost unchanged by the drug administration.

On the other hand, a significant action was not observed in the melatonin administration group.

From the result of this test, it was shown that compound A has an antidepressant effect and antianxiety effect.

### Example 4 (mouse tail suspension test)

Action on immobility and moving power in a single application test of Paroxetine and compound A
Used Animals: male ICR (Jcl) mice of 6 weeks old were used. Drug Administration Group and Test Method: Test was carried out for the following conditions. (each group n = 12)
1. Vehicle (0.5% methylcellulose)
2. Paroxetine 0.3 mg/kg
3. Paroxetine 1 mg/kg
4. Paroxetine 3 mg/kg
5. Paroxetine 10 mg/kg
6. Compound A 3 mg/kg
7. Compound A 10 mg/kg
8. Compound A 30 mg/kg

Paroxetine was dissolved in 0.5% methylcellulose physiological saline, and compound A was suspended in 0.5% methylcellulose aqueous solution for injection. To control group, a 0.5% methylcellulose physiological saline for injection was administered as vehicle. A dose of 20 ml/kg was administered intraperitoneally, respectively. Administration was carried out 30 minutes before trial of tail suspension.

Animals were put in a 5-row-cage with 8 each cage, and fully habituated. Drug administrations and trials were performed between 13 : 00 and 17 : 00.

### [Measurement Method]

An automated measuring equipment made according to the method of Steru (Psycopharmacology 85, 367-370, 1985) was used for measurement. The mouse was suspended by the tail in a soundproof box (16cm x 38cm x 33cm) divided individually, and then the immobility rate of 10 minutes duration was measured. Tail suspension was carried out by fixing a 5 cm wire to a sensor and suspending the mouse with fixing its tail with a tape at the end of the wire. By weighing every 10 msec, the movement of the mouse was calculated through A/D converter as moving power for one second. The moving power of less than 1% of body weight of mouse was considered to be in the immobile state, and the immobility rate for every one minute was measured. Test was carried out for 10 minutes, and all the experiments were controlled with personal computer (NEC-9801). The time course data of 5 minutes duration after trial and its average for 5 minutes were calculated, and showed in graphs.

### [Result]

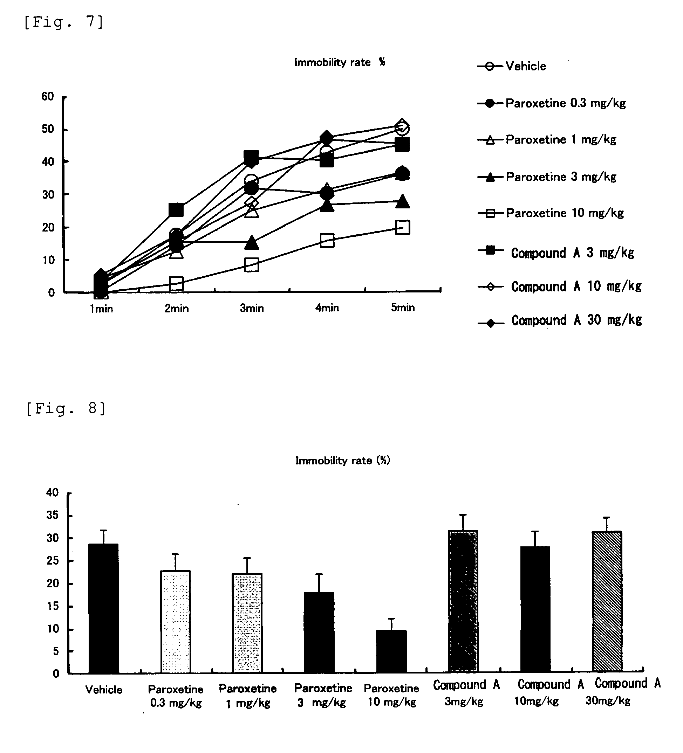

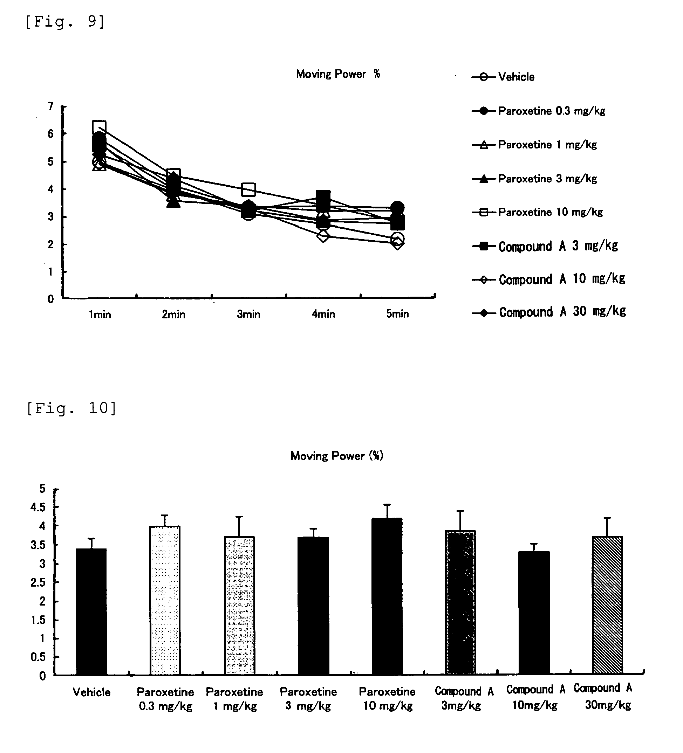

As shown in Figs. 7 to 10, a dose-dependent decrease in immobility was observed with the dose of 0.3, 1, 3 and 10 mg/kg (i.p.) of Paroxetine that is a SSRI, and antidepressant-like action was confirmed. But the action was not a significant action in Williams' test for multigroup. In addition, with respect to the moving power at the trial of tail suspension test, the tendency was observed to increase a little in Paroxetine administration groups compared to vehicle administration group. The dose of 3, 10 and 30 mg/kg (i.p.) of compound A didn't have an affect on the immobility and moving power at the trial of tail suspension test.

### Example 5 (mouse tail suspension test)

Action on immobility and moving power in a combined application test of Paroxetine and compound A
Used Animals: male ICR (Jcl) mice of 6 weeks old were used. Drug Administration Group and Test Method: Test was carried out for the following conditions. (each group n = 15-16)
1. Vehicle (0.5% methylcellulose)
2. Paroxetine 0.3 mg/kg
3. Paroxetine 1 mg/kg + Compound A 1 mg/kg
4. Paroxetine 1 mg/kg + Compound A 3 mg/kg
5. Paroxetine 1 mg/kg + Compound A 10 mg/kg

Paroxetine was dissolved in 0.5% methylcellulose physiological saline, and compound A was suspended in 0.5% methylcellulose aqueous solution for injection. To control group, a 0.5% methylcellulose physiological saline for injection was administered as vehicle. As a drug solution to be combined, a drug solution having twice concentration of final concentration was prepared, and adjusted to final concentration by mixing equal amounts. A dose of 20 ml/kg was administered intraperitoneally, respectively. Administration was carried out 30 minutes before trial of tail suspension.

Animals were put in a 5-row-cage with 8 each cage, and fully habituated. Drug administrations and trials were performed between 13 : 00 and 17 : 00.

### [Measurement Method]

An automated measuring equipment made according to the method of Steru (Psycopharmacology 85, 367-370, 1985) was used for measurement. The mouse was suspended by the tail in a soundproof box (16cm x 38cm x 33cm) divided individually, and then the immobility rate of 10 minutes duration was measured. Tail suspension was carried out by fixing a 5 cm wire to a sensor and suspending the mouse with fixing its tail with a tape at the end of the wire. By weighing every 10 msec, the movement of the mouse was calculated through A/D converter as moving power for one second. The moving power of less than 1% of body weight of mouse was considered to be in the immobile state, and the immobility rate for every one minute was measured. Test was carried out for 10 minutes, and all the experiments were controlled with personal computer (NEC-9801). The time course data of 5 minutes duration after trial and its average for 5 minutes were calculated, and showed in graphs.

### [Result]

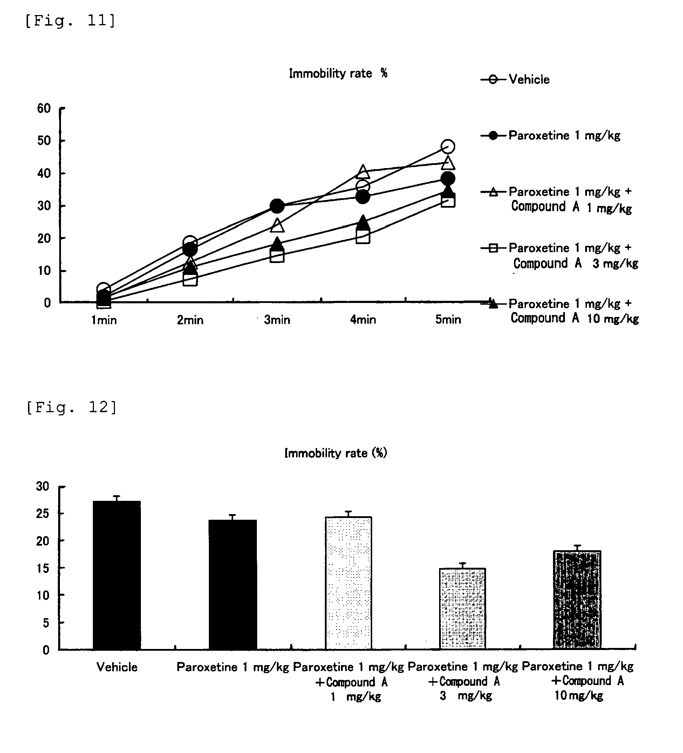

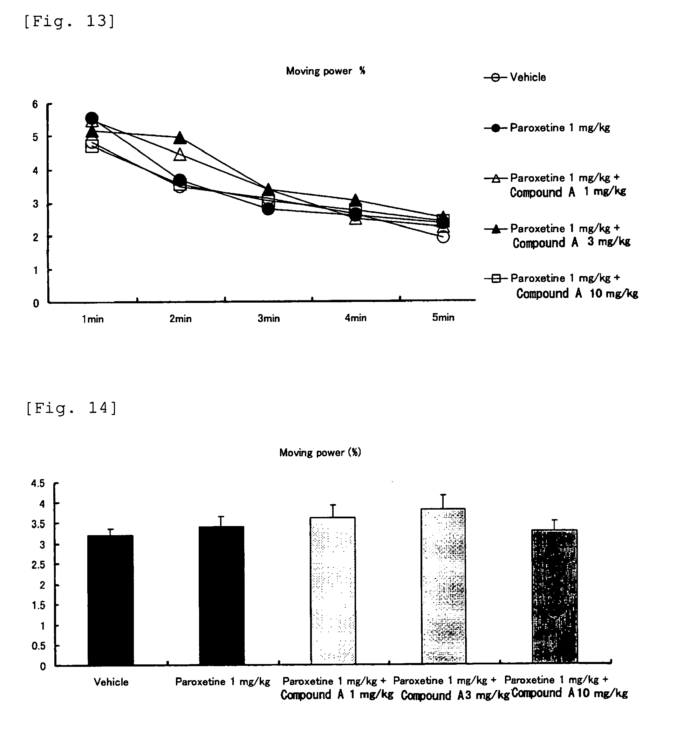

As shown in Figs. 11 to 14, a decrease in immobility rate was confirmed by the combined use of 1 mg/kg (i.p.) of Paroxetine and 1, 3 and 10 mg/kg of Compound A, and it was indicated that compound A enhances the antidepressant action of Paroxetine.

### Preparation Example

Compound A (160 g), lactose (4064 g), and corn starch (640 g) are mixed uniformly in a fluidized bed granulation dryer, and the mixture is granulated with spraying a solution of hydroxypropyl cellulose (160 g) in water in the dryer, followed by drying in said drier. The resulting granulated material is crushed by 1.5 mmφ punching screen using a power mill apparatus to obtain uniform granules. To the uniform granules (3894 g) are added corn starch (124 g) and magnesium stearate (12.4 g), and the mixture is mixed to give granules for tableting. These granules are tableted in a weight of 130 mg per tablet with a 7.0 mmφ die using a tableting machine to prepare bare tablets. The obtained bare tablets are sprayed with a solution of hydroxypropylmethylcellulose 2910 and copolividone wherein titanium oxide and yellow ferric oxide are dispersed, in a film coating machine, to give about 25000 tablets which are film-coated tablets each containing 4 mg of compound A per tablet and having a prescription shown in Table 1.

**[Table 1]**

| Composition | Blending Quantity (mg) |
|---|---|
| Compound A | 4.0 |
| Lactose | 101.6 |
| Corn Starch | 20.0 |
| Hydroxypropyl Cellulose | 4.0 |
| Magnesium stearate | 0.4 |
| Bare Tablet | 130.0 |
| Hydroxypropylmethylcellulose 2910 | 3.74 |
| Copolividone | 0.75 |
| Titanium Oxide | 0.5 |
| Yellow Ferric Oxide | 0.01 |
| Total | 135.0 |

### Industrial Applicability

According to the present invention, there are provided a prophylactic or therapeutic agent for depression or anxiety disorders, and the like.

## Claims

1. A pharmaceutical composition for prevention or treatment of depression or anxiety disorders, which comprises (S)-N-[2-(1,6,7,8-tetrahydro-2H-indeno[5,4-b]furan-8-yl)ethyl]propionamide.

2. A pharmaceutical composition for prevention or treatment of depression or anxiety disorders, which comprises (S)-N-[2-(1,6,7,8-tetrahydro-2H-indeno[5,4-b]furan-8-yl)ethyl]propionamide in combination with one or more drugs selected from other antidepressants and antianxiety drugs.

3. A pharmaceutical composition for prevention or treatment of depression or anxiety disorders, which comprises (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam.

4. The pharmaceutical composition for prevention or treatment of depression or anxiety disorders according to any one of claims 1 to 3, which is a prophylactic or therapeutic agent for depression or anxiety disorders of patients having a background of diabetes, hyperlipidemia, hypertension or metabolic syndrome.

5. A method for preventing or treating depression or anxiety disorders, which comprises administering (S)-N-[2-(1,6,7,8-tetrahydro-2H-indeno[5,4-b]furan-8-yl)ethyl]propionamide.

6. A method for preventing or treating depression or anxiety disorders of patients having a background of diabetes, hyperlipidemia, hypertension or metabolic syndrome, which comprises administering (S)-N-[2-(1,6,7,8-tetrahydro-2H-indeno[5,4-b]furan-8-yl)ethyl]propionamide.

7. A method for preventing or treating depression or anxiety disorders, which comprises administering (S)-N-[2-(1,6,7,8-tetrahydro-2H-indeno[5,4-b]furan-8-yl)ethyl]propionamide in combination with one or more drugs selected from other antidepressants and antianxiety drugs.

8. A method for preventing or treating depression or anxiety disorders of patients having a background of diabetes, hyperlipidemia, hypertension or metabolic syndrome, which comprises administering (S)-N-[2-(1,6,7,8-tetrahydro-2H-indeno[5,4-b]furan-8-yl)ethyl]propionamide in combination with one or more drugs selected from other antidepressants and antianxiety drugs.

9. A method for preventing or treating depression or anxiety disorders, which comprises administering (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam.

10. A method for preventing or treating depression or anxiety disorders of patients having a background of diabetes, hyperlipidemia, hypertension or metabolic syndrome, which comprises administering (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam.

11. Use of (S)-N-[2-(1,6,7,8-tetrahydro-2H-indeno[5,4-b]furan-8-yl)ethyl]propionamide for manufacturing a pharmaceutical composition for prevention or treatment of depression or anxiety disorders.

12. Use of (S)-N-[2-(1,6,7,8-tetrahydro-2H-indeno[5,4-b]furan-8-yl)ethyl]propionamide for manufacturing a pharmaceutical composition for prevention or treatment of depression or anxiety disorders of patients having a background of diabetes, hyperlipidemia, hypertension or metabolic syndrome.

13. Use of (S)-N-[2-(1,6,7,8-tetrahydro-2H-indeno[5,4-b]furan-8-yl)ethyl]propionamide in combination with one or more drugs selected from other antidepressants and antianxiety drugs for manufacturing a pharmaceutical composition for prevention or treatment of depression or anxiety disorders.

14. Use of (S)-N-[2-(1,6,7,8-tetrahydro-2H-indeno[5,4-b]furan-8-yl)ethyl]propionamide in combination with one or more drugs selected from other antidepressants and antianxiety drugs for manufacturing a pharmaceutical composition for prevention or treatment of depression or anxiety disorders of patients having a background of diabetes, hyperlipidemia, hypertension or metabolic syndrome.

15. Use of (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam for manufacturing a pharmaceutical composition for prevention or treatment of depression or anxiety disorders.

16. Use of (S)-N-[2-(1,6,7,8-tetrahydro-2H-indeno[5,4-b]furan-8-yl)ethyl]propionamide in combination with one or more drugs selected from Fluoxetine, Sertraline, Paroxetine, Mianserin, Milnacipran, Citalopram, Escitalopram, Fluvoxamine, Minaprine, Duloxetine, Venlafaxine, Imipramine, Clomipramine, Doxepin, Trazodone, Nefazodone, Amitriptyline, Carbamazepine, Mirtazapine, Diazepam, Flutazolam, Lorazepam, Buspirone, Tandospirone, Ethyl loflazepate, Flutoprazepam, Mexazolam, Clotiazepam, Etizolam, Hydroxyzine, Alprazolam, Fludiazepam, Chlordiazepoxide, Cloxazolam, Clorazepate and Oxazolam for manufacturing a pharmaceutical composition for prevention or treatment of depression or anxiety disorders of patients having a background of diabetes, hyperlipidemia, hypertension or metabolic syndrome.
